# EUROPEAN PATENT APPLICATION

(11) **EP 1 923 460 A1**
(43) Date of publication of application: **21.05.2008**
(21) Application number: 07254452.1
(22) Date of filing: 14.11.2007
(51) Int. Cl.: C12M 1/00, C12M 1/04

(54) **A bioreactor**

(30) Priority: 15.11.2006 US 859178 P
(71) Applicant: MILLIPORE CORPORATION, Billerica, Massachusetts 01821-3405 (US)
(72) Inventor: Belongia, Brett, Andover Massachusetts 01845 (US); Schauer, Neil, Milford Massachusetts 01757 (US)
(74) Representative: Greenwood, John David

(57) **Abstract**

A bioreactor formed of a flexible material is provided having a constant aspect ratio and an adjustable length.

## Description

### FIELD OF THE INVENTION

This invention relates to bioreactors.

### BACKGROUND OF THE INVENTION

The culture of microbial cells (fermentation) or animal and plant cells (tissue culture) are commercially-important chemical and biochemical production processes. Living cells are employed in these processes because living cells, using generally easily obtainable starting materials, can economically synthesize commercially-valuable chemicals including proteins such as monoclonal antibodies or enzymes; vaccines or alcoholic beverages.

Fermentation involves the growth or maintenance of living cells in a nutrient liquid media. In a typical batch fermentation process, the desired microorganism or eukaryotic cell is placed in a defined medium composed of water, nutrient chemicals and dissolved gases, and allowed to grow (or multiply) to a desired culture density. The liquid medium must contain all the chemicals which the cells require for their life processes and also should provide the optimal environmental conditions for their continued growth and/or replication. Currently, a representative microbial cell culture process might utilize either a continuous stirred-tank reactor or a gas-fluidized bed reactor in which the microbe population is suspended in circulating nutrient media. Similarly, in vitro mammalian cell culture might employ a suspended culture of cells in roller flasks or, for cells requiring surface attachment, cultures grown to confluence in tissue culture flasks containing nutrient medium above the attached cells. The living cells, so maintained, then metabolically produce the desired product(s) from precursor chemicals introduced into the nutrient mixture. The desired product(s) are either purified from the liquid medium or are extracted from the cells themselves.

At the present time, the biotechnology industry has traditionally utilized stainless steel bioreactors and piping in the manufacturing process since they can be sterilized and reused. However, these systems are costly. In addition, these systems require the periodic transfers of the cell cultures as they grow with an attendant reaction volume increase during the course of the bioreaction. However, the effective reaction volume of large reactor is not linearly scalable as the culture volume increases. As a result, mixing conditions will change due to an increase in culture volume and the culture will not be uniformly mixed. It is therefore, necessary to transfer the cell culture to a bioreactor having a different geometry in order to attain essentially the same mixing conditions. This procedure requires the maintenance of a multiplicity of reactor sets, usually three sets with consequent increase in capital costs. In addition, the cell culture transfer conditions must be maintained to prevent cell culture contamination. This requirement adds significantly to the bioreaction costs.

Within the linearly scalable reaction system employed, there must be included means to circulate the cell culture without dead zones within the reactor so as to effect complete bioreaction within the bioreactor. In addition, conditions under which the cells will shear must be avoided. Furthermore, means must be provided for adding nutrients, oxygen or carbon dioxide to maintain cell growth and cell viability as well as for maintaining proper desired pH. Also, care must be taken to initially sterilize and to subsequently exclude undesired cell types and cell toxins.

One system for a bioreactor has been to use a large table, equipped with motors or hydraulics onto which a bioreactor bag is placed. The motors/hydraulics rock the bag providing constant movement of the cells. Additionally, the bag has a gas and nutrient supply tube and a waste gas and waste product tube which allow for the supply of nutrients and gases such as air for aerobic organisms and the removal of waste such as respired gases, carbon dioxide and the like. The tubes are arranged to work with the motion of the bag to allow for a uniform movement of the gases and fluids/solids. See U.S. Pat. No. 6,190,913. Such a system requires the use of capital-intensive equipment, with components that are susceptible to wear. Additionally, the size of the bag that can be used with the table is limited by the size of table and the lifting capability of its motors/hydraulics.

An alternative system uses a long flexible tube-like bag that has both ends attached to movable arms such that the bag after filling is suspended downwardly from the movable arm in the shape of a U. The arms are then alternately moved upward or downward relative to the other so as to cause a rocking motion and fluid movement within the bag. If desired, the midsection may contain a restriction to cause a more intimate mixing action. This system requires the use of a specifically shaped bag and hydraulic or other lifting equipment to cause the movement of the liquid. Additionally, due to weight considerations, the bag size and volume is restricted by the lifting capacity of the equipment and the strength of the bag.

An improvement has been shown through the use of one or more bags that are capable of being selectively pressurized and deflated in conjunction with a disposable bio bag such as a fermenter, mixing bag, storage bag and the like. The pressure bag(s) may surround a selected outer portion of the bag or may be contained within an inner portion of such a bag. By selectively pressurizing and deflating the pressure bag(s), one is able to achieve fluid motion in the bag thereby ensuring cell suspension, mixing and/or gas and/or nutrient/excrement transfer within the bag without damaging shear forces or foam generation.

Alternatively, one can select a static (non-moving) bag that contains a sparger or other device for introducing a gas into the bag. The gas causes the movement of the fluid in the bag as well to cause the mixing and transfer of gases, nutrients and waste products.

U.S. Pat. No. 5,565,015 uses a flat, inflatable porous tube that is sealed into a plastic container. The tube inflates under gas pressure and allows gas to flow into the bag. When the gas is not applied, the tube collapses and substantially closes off the pores of the flat tube to prevent leakage from the bag.

U.S. Pat. No. 6,432,698 also inserts and seals a tube to a gas diffuser within the bag. It appears that a constant positive gas pressure must be maintained in order to prevent any liquid within the bag from entering the diffuser and then the gas line and eventually the air pump as no valve or other means for preventing backflow is shown.

Both of the structures disclosed by these two patents have the potential for leakage of the liquid in the container which can potentially contaminate the contents of the bag of the upstream components of the system such as the gas supply system. Additionally, both introduce a separate component for the gas distribution.

Accordingly, it would be desirable to provide a linearly scalable bioreactor apparatus and system which eliminates the need to transfer a cell culture from a first bioreactor to a second bioreactor. Such an apparatus and system would permit the use of a constant range of bioreaction conditions within one bioreactor.

### SUMMARY OF THE INVENTION

The present invention provides a disposable bioreactor which is linearly scaleable. By the term, "linearly scaleable" as used herein with reference to a bioreactor having a height, width and length is meant expandable in the length direction of the bioreactor while maintaining the aspect ratio (width/height) of the bioreactor constant. By maintaining the aspect ratio and cross sectional shape of the bioreactor constant and by increasing the length of the bioreactor over time, the mixing conditions within the bioreactor can be maintained essentially constant while increasing the effective volume of the bioreactor. This feature permits the use of one bioreactor over the full term of culture growth to produce the desired product(s).

The bioreactor includes means for introducing gas and for removing gas. The bioreactor also includes means for adding reactants and for removing desired product(s).

The bioreactor is formed of a flexible material such as a polymeric composition which can be folded upon itself, wound on itself or clamped on itself to form a seal. The flexible material does not contaminate the reactants or the products.

The bioreactor is shaped to affect movement of reactant liquid upwardly along an inner surface of an outer wall of the bioreactor and then downwardly within the reactant volume remote for the inner surface of the outer wall of the bioreactor.

The bioreactor includes a first inner surface of an outer wall which forms a closed volume with a second inner surface of an inner wall of the bioreactor. The first and second inner surfaces have at least a portion thereof which converge toward each other or diverge away from each other so that movement of reactant liquid within the bioreactor is in an essentially spiral direction under the influence of gases introduced into the bioreactor.

The bioreactor is also formed such that it has no horizontal or substantially horizontal surface upon which the cells can deposit This may be accomplished by either using a horizontal surface which has a gas supply that forms bubbles through it so that cells are pushed away from that surface or by using an angled inner wall of the reactor or both. Preferably the angled inner wall is substantially vertical.

Embodiments of the present invention can provide a disposable bioreactor which is linearly scaleable to any desired volume. More particularly, this invention relates to such a bioreactor wherein its length dimension can be increased while the other dimensions and aspect ratio (width / height) of the bioreactor remain the same in the volume of the reactor wherein bioreaction is affected to maintain constant fluid dynamics.

### BRIEF DESCRIPTION OF THE DRAWINGS

Embodiments of the invention will now be described, by way of example only, with reference to the accompanying drawings of which:
Figure 1 is a diagrammatic isometric view of a bioreactor according to the present invention;
Figure 2 is a diagrammatic illustration the steps of expanding the bioreactor of Figure 1;
Figure 3 is a diagrammatic cross sectional view of a bioreactor of which includes the width and height of the effective bioreaction volume;
Figure 4 is a diagrammatic isometric view of an alternative embodiment of the present invention;
Figure 5 is a diagrammatic isometric view illustrating the use of clamps with the illustrated embodiments of this invention;
Figure 6 is a diagrammatic isometric view of a further bioreactor of the present invention utilizing clamps;
Figure 7 is a diagrammatic cross sectional view of a further bioreactor of the present invention; and
Figure 8 is a diagrammatic cross sectional view of a further bioreactor of the present invention.

### DESCRIPTION OF SPECIFIC EMBODIMENTS

In accordance with this invention, a disposable, expandable bioreactor is provided having a constant aspect ratio and constant cross section which includes its height and width wherein the bioreactor's effective volume is increased by increasing its length. The bioreactor initially has a relatively small effective volume into which a cell culture, nutrients and one or more gases are introduced to effect a bioreaction therein. By the term "effective volume" as used herein is meant the bioreactor volume wherein reaction occurs. A portion of the bioreactor volume comprises a gas containing volume positioned above the effective volume. When it is desired to increase the bioreactor effective volume, an expandable portion of the bioreactor is expanded. The expansion is in a direction to increase the length of the bioreactor thereby to increase the effective volume of the bioreactor. Since the cross section including the width and height of the bioreactor is maintained constant over the course of the expansion, the reaction conditions can be maintained constant over the course of bioreactor expansion since the mixing conditions can be maintained constant. This is because the circulation of reactants caused by introducing gases is the same within the bioreactor cross section regardless of position on the length dimension.

The bioreactor length can be increased in any manner. Thus, the end of the bioreaction not in current use can be unfolded, or unwound. In addition, the unused portion of the bioreactor can be separated from the currently used effective volume by one or more clamps which can be removed in series to obtain a desired effective volume over time.

The internal volume of the bioreactor is shaped so that the reactants are satisfactorily mixed together in all portions of the effective volume. Thus, dead zones where little or no mixing occurs are avoided.

The bioreactor is shaped to affect movement of reactant liquid upwardly along an inner surface of an outer wall of the bioreactor and then downwardly within the reactant volume remote for the inner surface of the outer wall of the bioreactor.

The bioreactor includes a first inner surface of an outer wall which forms a closed volume with a second inner surface of an inner wall of the bioreactor. The first and second inner surfaces have at least a portion thereof which converge toward each other or diverge away from each other so that movement of reactant liquid within the bioreactor is in an essentially spiral direction under the influence of gases introduced into the bioreactor.

The bioreactor is also formed such that it has no horizontal or substantially horizontal surface upon which the cells can deposit This may be accomplished by either using a horizontal surface which has a gas supply that forms bubbles through it so that cells are pushed away from that surface or by using an angled inner wall of the reactor or both. Preferably the angled inner wall is substantially vertical.

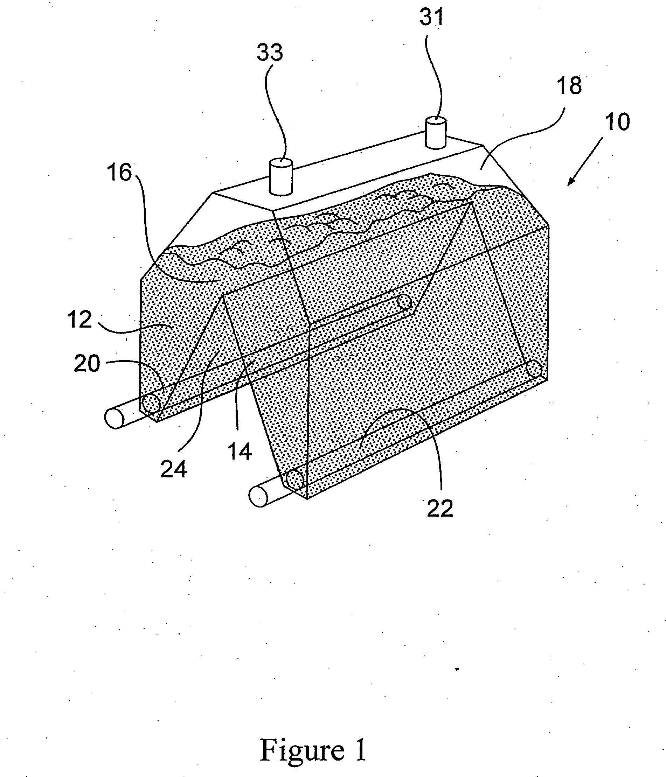

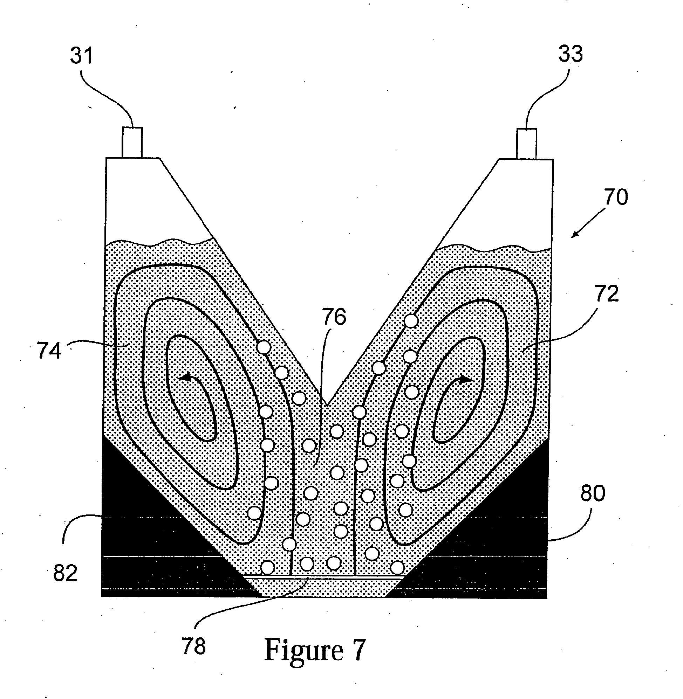

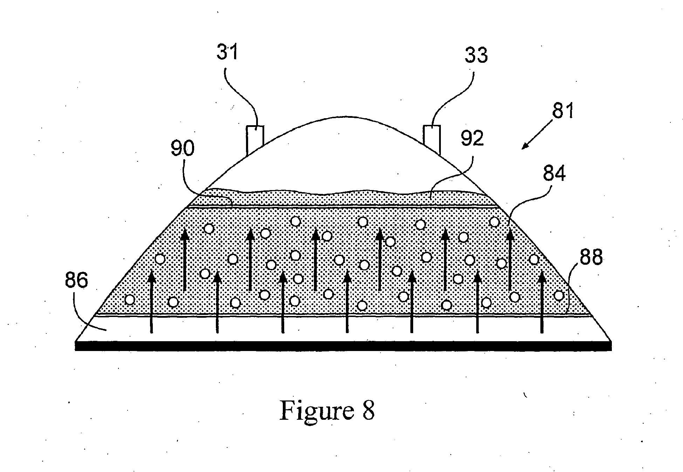

One embodiment of this design is a reactor having two legs connected to each other by a bridge section that is between the two legs where the two legs join such as is shown in Figures 1-7 or a rounded or ovular reactor wall shape as shown in Figure 8. Additionally the horizontal surface having the gas supply can be a porous filter or membrane as shown in Figures 7 and 8 or it may contain one or more spargers or other gas porous gas supply devices which pass the gas into the liquid as shown in Figures 1-6 and in both designs the gas either entrains the cells with its upward motion or it pushes the cells upward as it passes into the liquid.

A volume external the bioreactor is provided to house a heater which controls temperature within the bioreactor. One or more inlets to the bioreactor are provided for the purpose of introducing reactants into the bioreactor or to remove products from the bioreactor.

Gas is introduced into the effective volume of the bioreactor by at least one porous passage which can be formed integrally with the bioreactor such as by being adhered thereto along the length of the bioreactor. Alternatively, the porous passage(s) can be formed separately from the bioreactor such as a sparger tube and can be progressively inserted into the reactor when the effective volume of the reactor is increased. Conventional sealing means are provided to prevent leakage from the bioreactor at the areas where the porous passages are inserted into the reactor. The porous passages can be formed of a flexible material such as a polymeric composition which does not contaminate the reactants or product(s) or a rigid material such as a ceramic, a glass, such as a glass mat or a sintered glass material or sintered stainless steel which does not contaminate the reactants or product(s).

Suitable plastics can be hydrophilic or hydrophobic. When hydrophilic however one must ensure that the air pressure within the passage is either constantly at or above that of the liquid intrusion pressure so as to keep the liquid out of the passage or to provide an upstream shutoff such as a valve or hydrophobic filter to prevent the liquid in the bioreactor from flooding the passage and /or upstream gas supply. Plastics can be inherently hydrophilic or hydrophobic or can be surface treated to provide the desired properties. The plastics may be a single layer or if desired, multilayered. One example of a multilayered passage has a porous plastic layer covered by a more open prefilter or depth filter that can trap any debris and keep the debris from clogging the porous passage(s). The pore size or sizes selected depends upon the size of gas bubble desired. The pore size may range from microporous (0.1 to 10 microns) to macroporous (greater than 10 microns) and it may be formed of membranes or filters such as a microporous filter, woven fabrics or filters, porous non-woven materials, such as Tyvek® sheet materials, monoliths or pads, such as can be found in many aquarium filters and the like. The selected plastic(s) should be compatible with the bioreactor environment so it doesn't adversely affect the cells being grown within it. Suitable plastics include but are not limited to polyolefins such as polyethylene or polypropylene, polysulfones such as polysulfone or polyethersulfone, nylons, PTFE resin, PEF, PVDF, PET and the like.

The introduced gas functions both as a reactant and as a means for mixing the reactants.

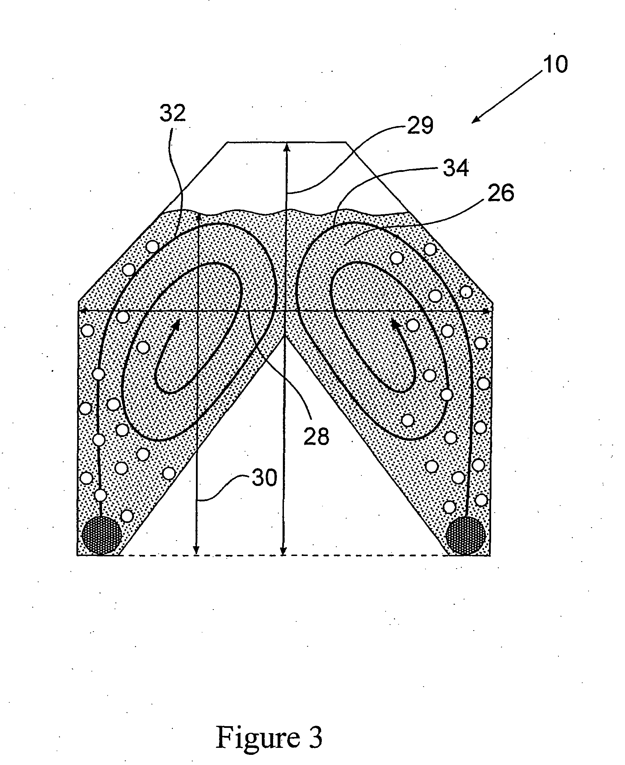

Referring to Figure 1, the bioreactor 10 includes two legs 12 and 14 which are connected by section 16 positioned above the legs 12 and 14. A gas volume 18 is provided above section 16 where unreacted introduced gas is collected. The gas is introduced into bioreactor 10 through passages 20 and 22 which are connected to a gas source (not shown). Inlets 31 and 33 are provided to introduce reactants, to remove product(s) or as gas vents to allow gases to escape. The external volume 24 is shaped to house a heater (not shown) for controlling the temperature within the bioreactor 10. As shown in Figure 3, the cross section of the internal volume 26 containing the width 28 of the effective volume and the height 29 is constant throughout the length of the bioreactor 10. Height 30 is the height of the effective volume which changes slightly with reactant addition or product removal. The height 30 of the effective volume can be maintained essentially constant by controlling the degree the effective volume is expanded, the volume of nutrients added and the volume of products removed. Thus, mixing conditions, as represented by arrows 32 and 34, are essentially constant throughout the length of the bioreactor 10 even after effective volume increase.

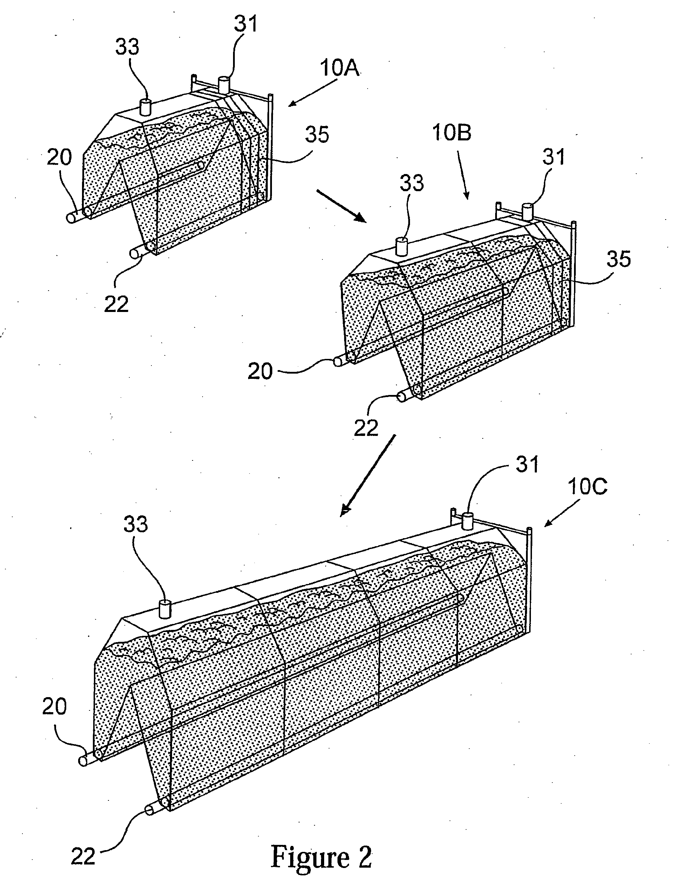

Referring to Figure 2, a sequence of bioreactor expansion steps is illustrated. In a first step, bioreactor 10 A is shown wherein a first step of the bioreaction is effected. A portion 35 of the bioreactor is folded upon itself. In a second step, a portion of the folded portion 35 is unfolded to expand the bioreactor 10 A along its length to form bioreactor 10 B wherein a second step of the bioreaction is effected. In a third step, the folded portion 35 is unfolded to expand the bioreactor 10 B along its length to form bioreactor 10 C wherein a third step of the bioreaction is effected. As shown, the cross section of the bioreactors containing the maximum width and height of the bioreactor 10 A, 10 B and 10 C remains constant with small changes, if any, due to reactant addition and/or product removal. The height of the effective volume remains essentially constant. Upon completion of the bioreaction, the bioreactor 10 C can be disposed.

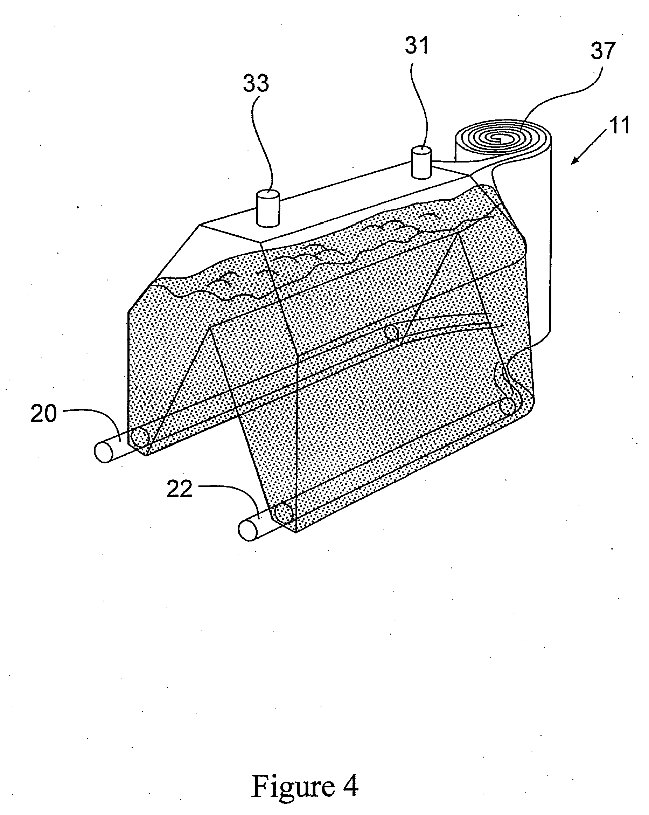

Referring to Figure 4, an alternative configuration of the bioreactor 11 of this invention is shown. The bioreactor 11 is constructed essentially the same as bioreactor 10 A (Figure 2) except that the unexpanded portion 37 is wound upon itself rather than being folded upon itself. The unexpanded portion 37 is unwound a desired length during the course of the desired bioreaction. In use, the bioreactor is utilized in the manner exemplified by the illustration of Figure 2 and, upon completion of the bioreaction and recovery of the products can be discarded at acceptable cost.

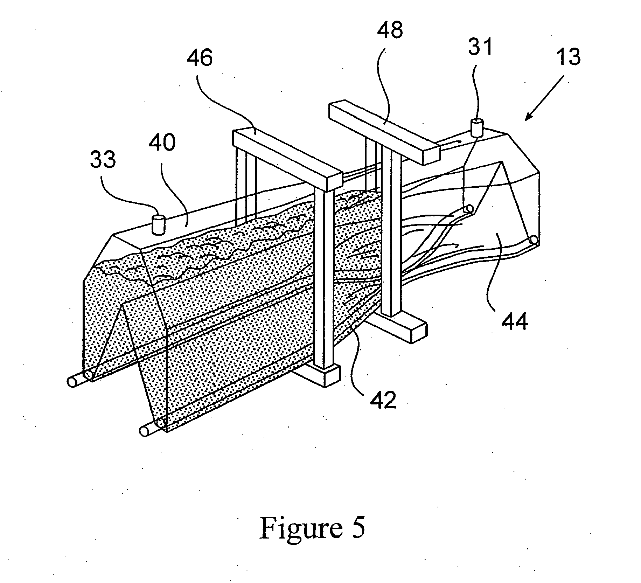

Referring to Figure 5, the bioreactor 13 having the same configuration as the bioreactor of Figure 1 is segmented into separate volumes 40, 42 and 44 by means of clamps 46 and 48. The inlets 31 and 33 are provided to introduce reactants, remove products or as gas vents to allow gases to escape. When it is desired to increase the effective volume of bioreactor 13, clamp 46 is released to combine volumes 40 and 42. When it is desired to further increase the effective volume of bioreactor 13, clamp 48 is released to combine volumes 40, 42 and 44. The bioreactor of Figure 5 permits the use of a more rapid process for effecting bio reaction. Initial bioreactions can be effected simultaneously in volumes 40 and 44 when clamps 46 and 48 are closed and the final bioreaction can be effected in volumes 40, 42 and 44 simultaneously after clamps 46 and 48 are released. Thus initial bioreactions can be effected in double the volume as compared with present bioreactors since double the volume of the bioreactor can be utilized initially at the desired reaction conditions and remaining volume(s) of the bioreactor can be utilized on a desired schedule.

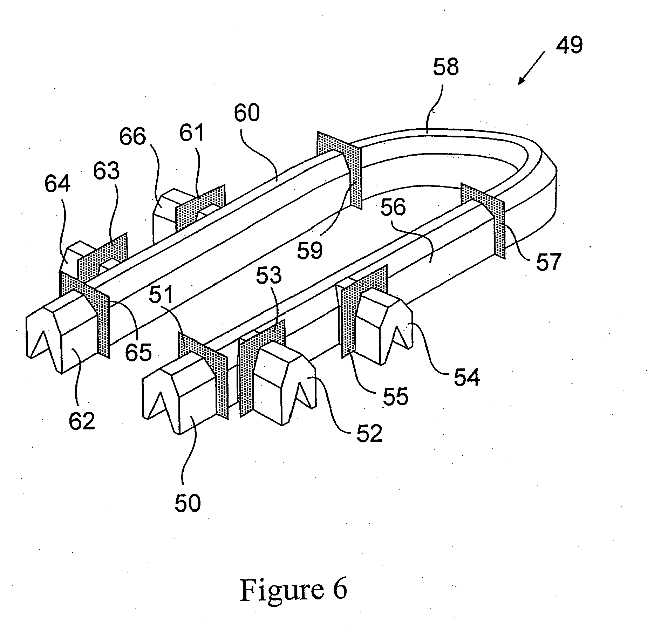

Referring to Figure 6, the bioreactor 49 includes volumes 50, 52, 54, 56, 58, 60, 62, 64 and 66 and clamps 51, 53, 55, 57, 59, 61, 63 and 65. Initial bio reactions are effected simultaneously in volumes 50, 52, 54, 62, 64 and 66. When it is desired to increase the effective volume of bioreactor 49, clamps 51, 53, 55 61, 63 and 65 are released to combine volumes 50, 52, 54 with volume 56 and to combine volumes 62, 64 and 66 with volume 60 to effect secondary bioreactions therein. When it is desired to further increase the effective volume of bioreactor 49, clamps 57 and 59 are released to combine all the volumes 50, 52, 54, 56, 58, 60, 62, 64 and 66. The bioreactor of Figure 6 permits the use of a more rapid process for effecting bioreaction since initial bioreactions can be effected simultaneously in six volumes which then can be combined with additional volumes sequentially as desired. Volumes 50, 52, 54, 62, 64 and 66 can be formed integrally with the remaining bioreactor volumes or separately therefrom. When formed separately, they can be sealed to the remaining volumes of the reactor such as with an adhesive or pneumatically.

Alternatively, the bioreaction can be effected sequentially by starting in one volume, such as volume 50 and then progress in size by opening one or more additional volumes 52, 54, 62, 64 and 66 sequentially as needed.

Referring to Figure 7, the bioreactor 70 is formed of a flexible material and has an expandable length in the manner described above with reference to Figures 1-6. The inlets 31 and 33 are provided to introduce reactants, remove products or as gas vents to allow gases to escape. The legs 72 and 74 are connected by section 76 positioned below legs 72 and 74. Gas is introduced through porous passage 78 positioned within section 76. Two heaters 80 and 82 control the temperature within bioreactor 70 and also provide support for bioreactor 70.

Referring to Figure 8, the bioreactor 81 is formed of a flexible material and has an expandable length in the manner described above with reference to Figures 1-6. The inlets 31 and 33 are provided to introduce reactants, remove products or as gas vents to allow gases to escape. The reactants are positioned within volume 84 positioned above volume 86 through which gas enters the bioreactor 81. The gas passes through gas permeable membrane 88 in a controlled manner so as to avoid rupturing the cells therein. A second membrane 90 is positioned below the surface 92 of the reactants so as to control gas passing therethrough in order to avoid foaming of the reactants and to avoid rupturing the cells.

The bioreactor of this invention can be formed of a flexible plastic material. Preferably thermoplastics are used and include but are not limited, polyolefins homopolymers such as polyethylene and polypropylene, polyolefins copolymers, nylons, ethylene vinyl acetate copolymers (EVA copolymers), ethylene vinyl alcohols (EVOH) and the like. Multilayered films or sheets are preferably used as the bioreactor materials and are generally made of several layers of polyethylene or polypropylene, such as linear low density polyethylene with other layers such as ethylene vinyl acetate copolymers and ethylene vinyl alcohols that are used to adhere layers together and/or to block gas transfer out of the bioreactor. It is preferred that the plastic be transparent so the activity within can be conducted by visual inspection.

## Claims

1. A bioreactor formed of a flexible material having a constant aspect ratio and an adjustable length.

2. A bioreactor formed of a flexible material, having an internal volume including two leg sections joined by a bridge section, means for introducing gas into each of said leg sections, means for introducing gas into said leg sections along the length of said leg sections, means for introducing reactants into said bioreactor, means for removing product from said bioreactor, said bioreactor having a constant aspect ratio and an adjustable length.

3. The bioreactor of Claim 1 or 2 wherein said length is adjusted by unfolding a length of folded bioreactor.

4. The bioreactor of Claim 1 or 2wherein said length is adjusted by unwinding a length of wound bioreactor.

5. The bioreactor of Claim 1 or 2 wherein said length is adjusted by releasing one or a plurality of clamps positioned along the length of said bioreactor.

6. The bioreactor of Claim 2 wherein said bridge section is positioned above said leg sections.

7. The bioreactor of Claim 2 wherein said bridge section is positioned below said leg sections.

8. The bioreactor of any preceding claim wherein the bioreactor is formed such that it has no horizontal or substantially horizontal surface upon which the cells can deposit.

9. The bioreactor of Claim 8 bioreactor is in a rounded shape.

10. The bioreactor of Claim 7 wherein the means for introducing the gas is a porous passage positioned within bridge.

11. The bioreactor of any preceding claim wherein the bioreactor further comprises a constant cross section.

12. The bioreactor of any preceding claim wherein the bioreactor cross section is maintained constant over the course of the length.

13. The bioreactor of any preceding claim wherein a volume external the bioreactor is provided to house a heater.

14. The bioreactor of any preceding claim wherein the means for introducing gas is a porous passage formed of one or more layers of porous material.
